# EUROPEAN PATENT SPECIFICATION

(11) **EP 4 260 893 B1**
(45) Date of publication and mention of the grant of the patent: **30.10.2024**
(21) Application number: 22168591.0
(22) Date of filing: 14.04.2022
(51) Int. Cl.: A61M 16/00, A61M 16/10, A61M 16/16, A61M 11/00, A61M 16/18

(54) **ACTIVE HUMIDIFICATION DEVICE**
AKTIVE BEFEUCHTUNGSVORRICHTUNG
DISPOSITIF D'HUMIDIFICATION ACTIF

(43) Date of publication of application: 18.10.2023
(73) Proprietor: Sedana Medical Limited, Kildare Two Mile House, Naas (IE)
(72) Inventor: HENNESSY, Harry, 112 46 Kungsholmen, Stockholm (SE); NARAYANAN, Vishnu, 11415 Stockholm (SE)
(74) Representative: Schütte, Gearoid

(56) References cited:
- EP-A1- 3 160 559
- WO-A1-2012/077052
- WO-A1-2018/035579
- WO-A1-2020/240019
- US-A- 6 102 037
- US-A1- 2013 263 851
- US-A1- 2017 000 968
- US-A1- 2018 250 490
- US-B2- 9 375 548

## Description

### Introduction

This invention relates to an active humidification device for a patient breathing circuit.

### Background to the invention

It is known to provide heat and moisture reflecting devices, such as a HME (Heat and Moisture Exchanger) or a sedation device of the type described in our earlier patent application - publication no. WO 2017/220698 A1, in a patient breathing circuit. These devices act as a passive humidifier, where a percentage of the heat and moisture in air exhaled by a patient is reflected back to the patient (approximately 32°C and 33mg moisture/litre of air). However, it is often desirable to maintain an optimal air temperature and humidity of 37°C and 44mg/l for inhalation by the patient. To achieve this optimum air condition, it has previously been proposed to mount an active humidification device in the patient breathing circuit - see for example WO 2009/118718, EP 3 160 559 and US 9,375,548 B2. An active and passive humidification device for mounting in a patient ventilation circuit is disclosed in WO 2020/240019A1. Examples of respiratory humidification systems are disclosed in US 6,102,037A and US 2018/250490A1. Other humidification systems and devices are disclosed in WO 2012/077052A1, US 2013/0263851A1, US 2017/000968A1 and WO 2018/035579A1.

The present invention is directed towards providing an improved active humidification device for mounting in a patient breathing circuit.

### Summary of the invention

According to the invention, there is provided an active humidification device for a patient breathing circuit, comprising:
a housing,
an air conditioning chamber extending through the housing between a first connector port for connection to a ventilator and a second connector port for connection via a breathing tube to a patient,
a heater mounted within the air conditioning chamber,
a temperature and humidity sensor mounted within the air conditioning chamber,
an air flow sensor mounted within the air conditioning chamber, the air flow sensor operable to measure air flow rate and direction of air flow through the air conditioning chamber,
a humidity generator mounted on the housing and having a vapour outlet communicating with the air conditioning chamber, and
a controller operably connected to the heater, the temperature and humidity sensor, the humidity generator and the air flow sensor for controlling operation of the active humidification device to maintain air delivered from the active humidification device to a patient in use at a preset desired humidity, characterised in that the heater comprises an electro-resistive heater element extending around a cylindrical support sleeve defining an inner bore of the air conditioning chamber, with an outer insulation sleeve mounted about the heater element.

In one embodiment of the invention, the controller in response to the air flow sensor regulates operation of the humidity generator to discharge humidifying vapour into the air conditioning chamber only during inhalation by the patient.

In another embodiment, the air flow sensor is mounted at an inner end on the housing and projecting into the air conditioning chamber.

In another embodiment, the heater is mounted within the air conditioning chamber between the humidity generator and the second connector port.

In another embodiment, the temperature and humidity sensor is mounted between the heater and the second connector port.

In another embodiment, the air flow sensor is mounted between the humidity generator and the first connector port.

In another embodiment, the humidity generator comprises a piezo electric mesh nebulizer.

In another embodiment, the humidity generator has a water bowl having an inlet for connection to a water supply and an outlet for connection to a nebulizer which is operable to discharge water vapour into the air conditioning chamber.

In another embodiment, an air permeable filter is mounted between the air flow sensor and the humidity generator.

### Brief Description of the Drawings

The invention will be more clearly understood by the following description of some embodiments thereof, given by way of example only, with reference to the accompanying drawings, in which:

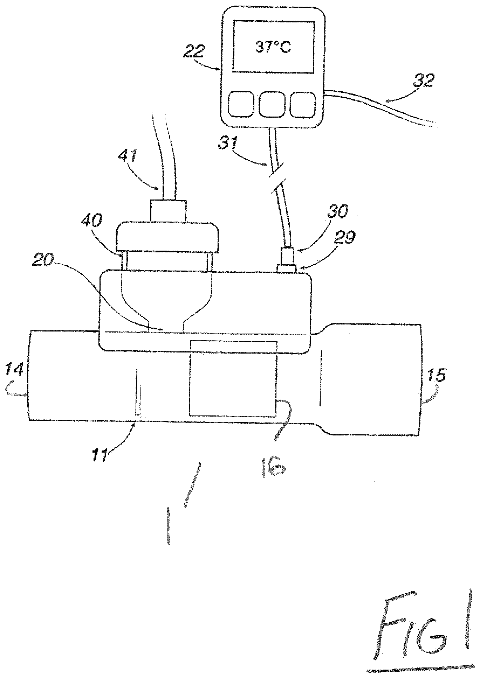

Fig. 1 is a schematic elevational view of an active humidification device according to the invention;

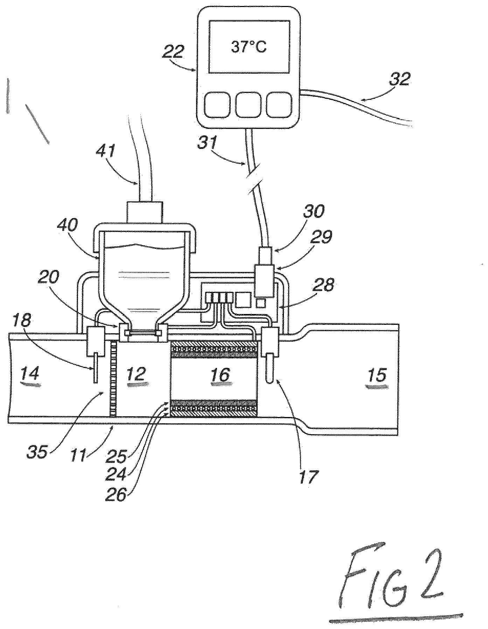

Fig. 2 is a schematic sectional elevational view of the active humidification device;
Fig. 3 is a schematic illustration showing the active humidification device in use; and
Fig. 4 is a schematic illustration showing the active humidification device in use.

### Detailed Description of the Preferred Embodiments

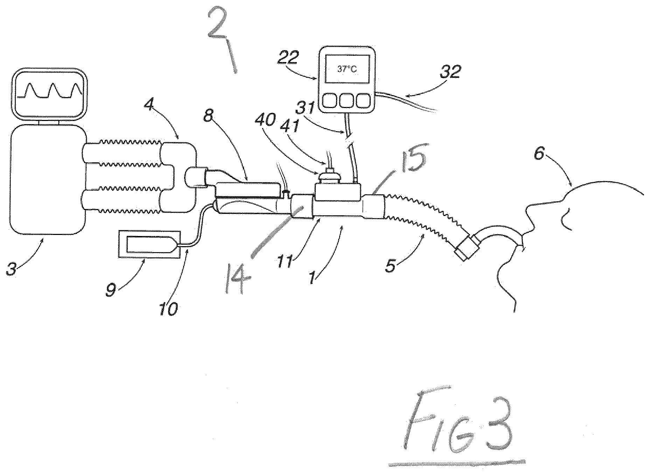

Referring to the drawings, there is illustrated an active humidification device according to the invention, indicated generally by the reference numeral 1. The active humidification device 1 is for mounting in a patient breathing circuit, indicated generally by the reference numeral 2 (Fig. 3), which typically includes a ventilator 3 connected by a Y-piece 4 with an elongate patient breathing tube 5 for a patient 6. A HME device, or in this case a sedation device 8, of the type described in WO 2017/220698 A1, may be mounted in the patient breathing tube 5 between the ventilator 3 and the patient 6 to passively reflect heat and moisture to the patient 6 as the patient 6 breathes. A sedative reservoir 9 of the sedation device 8 may also supply a sedative via a sedative delivery line 10 through the sedation device 8 to the patient 6 as described in WO 2017/220698 A1. The active humidification device 1 of this invention is mounted in the patient breathing tube 5 between the sedation device 8 and the patient 6, and is operable to maintain breathing air delivered to the patient 6 at a pre-set desired temperature and humidity, for example one specific treatment protocol may require a temperature of 37°C and 100% relative humidity (44 mg H₂O/l).

The active humidification device 1 comprises a housing 11 having an air conditioning chamber 12 extending through the housing 11 between a first connector port 14, for connection via the patient breathing tube 5 with the ventilator 3, and a second connector port 15, for connection via the patient breathing tube 5 with the patient 6 in use.

A heater 16 is mounted within the air conditioning chamber 12 and a temperature and humidity sensor 17 is mounted at a top of the air conditioning chamber 12. Also mounted within the air conditioning chamber 12 is an air flow sensor 18 which is operable to measure air flow rate and the direction of air flowing through the air conditioning chamber 12.

A humidity generator, in this case comprising a nebuliser 20, is mounted on the housing 11 and has a water vapour outlet communicating with the air conditioning chamber 12. It will be appreciated that other types of humidity generator, such as a heated wick for example, could be provided instead of the nebulizer 20.

A controller 22 is operably connected to the heater 16, the temperature and humidity sensor 17, the air flow sensor 18 and the nebuliser 20 for controlling operation of the active humidification device 1 to maintain air delivered from the active humidification device 1 to the patient 6 in use at the pre-set desired temperature and relative humidity. The controller is operable for adjusting the temperature and humidity for different treatment protocols and other procedures.

The heater 16 comprises a generally cylindrical electro-resistive heater element 24 wrapped around a cylindrical steel support sleeve 25 which defines an inner bore of the air conditioning chamber 12. An outer insulation sleeve 26 is mounted about the heater element 24 to prevent the housing 11 heating up and burning medical staff or the patient 6. The temperature and humidity sensor 17 is mounted at a top of the air conditioning chamber 12 on a power input board 28 within the housing 11 at a top of the housing 11, and is located between the heater 16 and the second connector port 15 leading to the patient 6. The temperature and humidity sensor 17 measures the air temperature within the air conditioning chamber 12 and communicates with the controller 22 which in turn provides the correct power to the heater element 24 to maintain air at a steady temperature of 37°C.

A connector socket 29 on the power input board 28 is engagable by a plug connector 30 which connects via cable 31 with the controller 22. The controller 22 connects via power cable 32 with a power supply. The controller 22 communicates with, controls and powers all components 16, 17, 18, 20 within the active humidification device 1.

Optionally, an air permeable filter 35 is mounted across the air conditioning chamber 12 between the nebuliser 20 and the air flow sensor 18 to block any secretions that might adversely affect the flow sensor 18 functionality.

The nebuliser 20 has a water bowl 40 which connects by a water feed line 41 with a water supply (not shown), which could be a water reservoir tank, bottle or pouch for example, and conveniently may be gravity fed to the water bowl 40. The nebuliser 20 is a piezoelectric mesh nebuliser 20 which discharges water vapour as required into the air conditioning chamber 12 to maintain the required humidity. It will be noted that the nebuliser 20 is controlled by the controller 22 to generate a mist of water vapour only during inhalation by the patient 6 to prevent clogging of filters in the active humidification device 1 and the associated HME or sedation device 8 during exhalation. In this regard the flow sensor 18 detects the direction of air flow through the air conditioning chamber 12 so the controller 22 can determine if the patient 6 is inhaling or exhaling and synchronise nebuliser 20 operation with patient 6 inhalation. The flow sensor 18 also measures the rate of air flow, this measurement being used by the controller 22 to vary the output of the nebuliser 20, as the amount of added moisture required to increase the humidity to 100% is proportional to the rate of air flow.

In use, the active humidification device 1 is mounted in the patient breathing circuit 2 between the sedation device 8 and the patient 6. A desired temperature and humidity is pre-set on the controller 22, which will regulate operation of the active humidification device 1 to maintain the pre-set temperature and humidity in the air breathed in by the patient 6. The temperature of air exhaled by the patient 6 into the air conditioning chamber 12 is measured by the temperature and humidity sensor 17 and together with the air flow rate measured by the air flow sensor 18 is used by the controller 22 to operate the heater 16 and the nebuliser 20 to bring the air to the required pre-set temperature and humidity for the next inhalation by the patient 6.

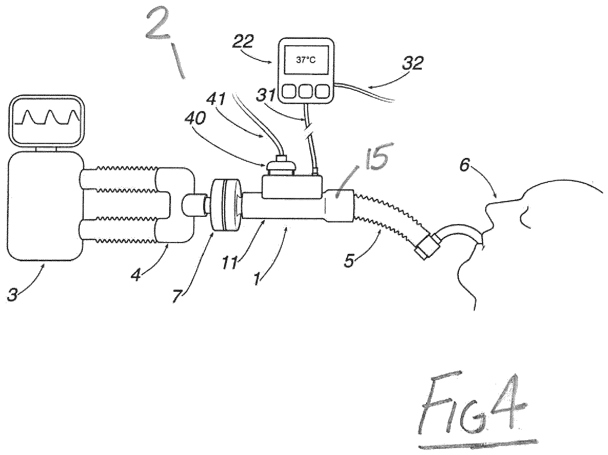

Referring to Fig. 4, the active humidification device 1 of the invention is shown in use in a patient breathing circuit 2. In this case, instead of the sedation device 8 shown in Fig. 3, a HME (heat and moisture exchange) device 7 is mounted in the patient breathing circuit 2, between the active humidification device 1 and the ventilator 3.

It is envisioned that in a specific treatment protocol the pre-set temperature and humidity will be a temperature of 37°C and 100% relative humidity (44 mg H₂O/l). However, it will be appreciated that the required temperature and humidity can be adjusted by means of the controller 22, as required. For example, it may at times be desirable to drop the humidity and dry out the circuit.

The terms "comprise" and "include", and any variations thereof required for grammatical reasons, are to be considered as interchangeable and accorded the widest possible interpretation.

The invention is not limited to the embodiments hereinbefore described which may be varied in both construction and detail within the scope of the appended claims.

## Claims

1. Active humidification device (1) for a patient breathing circuit (2), comprising:
a housing (11),
an air conditioning chamber (12) extending through the housing (11) between a first connector port (14) for connection to a ventilator (3) and a second connector port (15) for connection via a breathing tube (5) to a patient (6),
a heater (16) mounted within the air conditioning chamber (12),
a temperature and humidity sensor (17) mounted within the air conditioning chamber (12),
an air flow sensor (18) mounted within the air conditioning chamber (12),
the air flow sensor (18) operable to measure air flow rate and direction of air flow through the air conditioning chamber (12),
a humidity generator (20) mounted on the housing (11) and having a vapour outlet communicating with the air conditioning chamber (12), and
a controller (22) operably connected to the heater (16), the temperature and humidity sensor (17), the air flow sensor (18) and the humidity generator (20) for controlling operation of the active humidification device (1) to maintain air delivered from the active humidification device (1) to a patient (6) in use at a preset desired humidity,
**characterised in that** the heater (16) comprises an electro-resistive heater element (24) extending around a cylindrical support sleeve (25) defining an inner bore of the air conditioning chamber (12), with an outer insulation sleeve (26) mounted about the heater element (24).

2. The active humidification device (1) as claimed in claim 1, wherein the controller (22) in response to the air flow sensor (18) regulates operation of the humidity generator (20) to discharge humidifying vapour into the air conditioning chamber (12) only during inhalation by the patient (6).

3. The active humidification device (1) as claimed in claim 1 or claim 2, wherein the air flow sensor (18) is mounted at an inner end on the housing (11) and projecting into the air conditioning chamber (12).

4. The active humidification device (1) as claimed in any preceding claim, wherein the heater (16) is mounted within the air conditioning chamber (12) between the humidity generator (20) and the second connector port (15).

5. The active humidification device (1) as claimed in any preceding claim, wherein the temperature and humidity sensor (17) is mounted between the heater (16) and the second connector port (15).

6. The active humidification device (1) as claimed in any preceding claim, wherein the air flow sensor (18) is mounted between the humidity generator (20) and the first connector port (14).

7. The active humidification device (1) as claimed in any preceding claim, wherein the humidity generator (20) comprises a piezo electric mesh nebulizer (20) .

8. The active humidification device (1) as claimed in any preceding claim, wherein the humidity generator (20) has a water bowl (40) having an inlet for connection to a water supply and an outlet for connection to a nebulizer (20) which is operable to discharge water vapour into the air conditioning chamber (12).

9. The active humidification device (1) as claimed in any preceding claim, wherein an air permeable filter (35) is mounted between the air flow sensor (18) and the humidity generator (20).

## Patentansprüche

1. Aktive Befeuchtungsvorrichtung (1) für einen Patientenbeatmungskreislauf (2), umfassend:
ein Gehäuse (11),
eine Klimakammer (12), die sich durch das Gehäuse (11) zwischen einem ersten Verbinderanschluss (14) zum Verbinden mit einem Beatmungsgerät (3) und einem zweiten Verbinderanschluss (15) zum Verbinden über einen Beatmungsschlauch (5) mit einem Patienten (6) erstreckt,
ein Heizelement (16), das innerhalb der Klimakammer (12) befestigt ist,
einen Temperatur- und Feuchtigkeitssensor (17), der innerhalb der Klimakammer (12) befestigt ist,
einen Luftströmungssensor (18), der innerhalb der Klimakammer (12) befestigt ist, wobei der Luftströmungssensor (18) betriebsfähig ist, um die Luftströmungsrate und eine Richtung der Luftströmung durch die Klimakammer (12) zu messen,
einen Feuchtigkeitsgenerator (20), der an dem Gehäuse (11) befestigt ist und einen Dampfauslass aufweist, der mit der Klimakammer (12) in Kommunikation steht, und
eine Steuerung (22), die mit dem Heizelement (16), dem Temperatur- und Feuchtigkeitssensor (17), dem Luftströmungssensor (18) und dem Feuchtigkeitsgenerator (20) zum Steuern der aktiven Befeuchtungsvorrichtung (1) betriebsfähig verbunden ist, um Luft, die von der aktiven Befeuchtungsvorrichtung (1) an einen Patienten (6) abgegeben wird, in Verwendung auf einer voreingestellten gewünschten Feuchtigkeit zu halten,
**dadurch gekennzeichnet, dass** das Heizelement (16) ein elektroresistives Heizelement (24) umfasst, das sich um eine zylindrische Trägerhülse (25) herum erstreckt, die eine Innenbohrung der Klimakammer (12) definiert, wobei eine äußere Isolierhülse (26) um das Heizelement (24) befestigt ist.

2. Aktive Befeuchtungsvorrichtung (1) nach Anspruch 1, wobei die Steuerung (22) als Reaktion auf den Luftströmungssensor (18) den Betrieb des Feuchtigkeitsgenerators (20) reguliert, um Befeuchtungsdampf in die Klimakammer (12) nur während einer Inhalation durch den Patienten (6) abzuführen.

3. Aktive Befeuchtungsvorrichtung (1) nach Anspruch 1 oder 2, wobei der Luftströmungssensor (18) an einem inneren Ende an dem Gehäuse (11) befestigt ist und in die Klimakammer (12) hineinragt.

4. Aktive Befeuchtungsvorrichtung (1) nach einem der vorstehenden Ansprüche, wobei das Heizelement (16) innerhalb der Klimakammer (12) zwischen dem Feuchtigkeitsgenerator (20) und dem zweiten Verbinderanschluss (15) befestigt ist.

5. Aktive Befeuchtungsvorrichtung (1) nach einem der vorstehenden Ansprüche, wobei der Temperatur- und Feuchtigkeitssensor (17) zwischen dem Heizelement (16) und dem zweiten Verbinderanschluss (15) befestigt ist.

6. Aktive Befeuchtungsvorrichtung (1) nach einem der vorstehenden Ansprüche, wobei der Luftströmungssensor (18) zwischen dem Feuchtigkeitsgenerator (20) und dem ersten Verbinderanschluss (14) befestigt ist.

7. Aktive Befeuchtungsvorrichtung (1) nach einem der vorstehenden Ansprüche, wobei der Feuchtigkeitsgenerator (20) einen piezoelektrischen Mesh-Vernebler (20) umfasst.

8. Aktive Befeuchtungsvorrichtung (1) nach einem der vorstehenden Ansprüche, wobei der Feuchtigkeitsgenerator (20) eine Wasserschüssel (40) aufweist, die einen Einlass für die Verbindung an eine Wasserversorgung und einen Auslass für die Verbindung an einen Vernebler (20), der betriebsfähig ist, um Wasserdampf in die Klimakammer (12) abzugeben, aufweist.

9. Aktive Befeuchtungsvorrichtung (1) nach einem der vorstehenden Ansprüche, wobei ein luftdurchlässiger Filter (35) zwischen dem Luftströmungssensor (18) und dem Feuchtigkeitsgenerator (20) befestigt ist.

## Revendications

1. Dispositif d'humidification active (1) destiné à un circuit respiratoire de patient (2), comprenant :
un logement (11),
une chambre de conditionnement d'air (12) s'étendant à travers le logement (11) entre un premier port de connecteur (14) pour raccordement à un respirateur (3) et un second port de connecteur (15) pour raccordement par l'intermédiaire d'un tube respiratoire (5) à un patient (6),
un chauffage (16) monté au sein de la chambre de conditionnement d'air (12),
un capteur de température et d'humidité (17) monté au sein de la chambre de conditionnement d'air (12),
un capteur d'écoulement d'air (18) monté au sein de la chambre de conditionnement d'air (12), le capteur d'écoulement d'air (18) fonctionnel pour mesurer un débit d'écoulement d'air et une direction d'écoulement d'air à travers la chambre de conditionnement d'air (12),
un générateur d'humidité (20) monté sur le logement (11) et ayant une sortie de vapeur communiquant avec la chambre de conditionnement d'air (12), et
un organe de commande (22) connecté fonctionnellement au chauffage (16), au capteur de température et d'humidité (17), au capteur d'écoulement d'air (18) et au générateur d'humidité (20) permettant de commander le fonctionnement du dispositif d'humidification active (1) pour maintenir l'air distribué du dispositif d'humidification active (1) à un patient (6) en cours d'utilisation à une humidité souhaitée préréglée,
**caractérisé en ce que** le chauffage (16) comprend un élément chauffant électro-résistif (24) s'étendant autour d'un manchon de support cylindrique (25) définissant un alésage interne de la chambre de conditionnement d'air (12), avec un manchon d'isolation externe (26) monté autour de l'élément chauffant (24).

2. Dispositif d'humidification active (1) selon la revendication 1, dans lequel l'organe de commande (22) en réponse au capteur d'écoulement d'air (18) régule le fonctionnement du générateur d'humidité (20) pour décharger de la vapeur d'humidification dans la chambre de conditionnement d'air (12) seulement pendant une inhalation par le patient (6).

3. Dispositif d'humidification active (1) selon la revendication 1 ou la revendication 2, dans lequel le capteur d'écoulement d'air (18) est monté au niveau d'une extrémité interne sur le logement (11) et fait saillie dans la chambre de conditionnement d'air (12).

4. Dispositif d'humidification active (1) selon l'une quelconque revendication précédente, dans lequel le chauffage (16) est monté au sein de la chambre de conditionnement d'air (12) entre le générateur d'humidité (20) et le second port de connecteur (15).

5. Dispositif d'humidification active (1) selon l'une quelconque revendication précédente, dans lequel le capteur de température et d'humidité (17) est monté entre le chauffage (16) et le second port de connecteur (15).

6. Dispositif d'humidification active (1) selon l'une quelconque revendication précédente, dans lequel le capteur d'écoulement d'air (18) est monté entre le générateur d'humidité (20) et le premier port de connecteur (14).

7. Dispositif d'humidification active (1) selon l'une quelconque revendication précédente, dans lequel le générateur d'humidité (20) comprend un nébuliseur piézoélectrique à mailles (20).

8. Dispositif d'humidification active (1) selon l'une quelconque revendication précédente, dans lequel le générateur d'humidité (20) a une cuve d'eau (40) ayant une entrée pour raccordement à une alimentation en eau et une sortie pour raccordement à un nébuliseur (20) qui est fonctionnel pour décharger de la vapeur d'eau dans la chambre de conditionnement d'air (12).

9. Dispositif d'humidification active (1) selon l'une quelconque revendication précédente, dans lequel un filtre perméable à l'air (35) est monté entre le capteur d'écoulement d'air (18) et le générateur d'humidité (20).
